# EUROPÄISCHE PATENTSCHRIFT

(11) **EP 1 681 283 B1**
(45) Veröffentlichungstag und Bekanntmachung des Hinweises auf die Patenterteilung: **08.09.2010**
(21) Anmeldenummer: 05028459.5
(22) Anmeldetag: 24.12.2005
(51) Int. Cl.: C07C 69/736, C07C 69/734, A61K 6/00

(54) **Hydrolysestabile Monomere mit Säuregruppen**
Acid groups containing hydrolytically stable monomers
Monomères stable à hydrolyse ayant des groupes d'acide

(30) Priorität: 17.01.2005 DE 102005002330
(43) Veröffentlichungstag der Anmeldung: 19.07.2006
(73) Patentinhaber: Heraeus Kulzer GmbH, 63450 Hanau (DE)
(72) Erfinder: Hoffmann, Marcus, Dr., 61250 Usingen (DE); Erdrich, Albert, Dr., 61231 Bad Nauheim (DE)
(74) Vertreter: Kühn, Hans-Christian

(56) Entgegenhaltungen:
- EP-A- 1 421 927
- US-A- 4 755 620
- US-A1- 2003 055 124
- US-B1- 6 172 131

## Beschreibung

Die Erfindung betrifft hydrolysestabile Monomere mit Säuregruppen, deren Herstellung und Verwendung.

Seit wenigen Jahren sind selbstätzende Adhäsive auf dem Dentalgebiet bekannt. Sie vereinigen einen Ätz- und einen Bonding-Schritt, so dass die Oberfläche für die Polymer- bzw. Kompositfüllung präpariert ist. Es handelt sich in der Regel um zweikomponentige Materialien, die entweder direkt vor der Anwendung gemischt oder nacheinander aufgetragen werden müssen. Eines der ersten einkomponentigen Einflaschenadhäsive, bei dem kein Anmischen oder aufeinanderfolgendes Auftragen zweier Komponenten erforderlich ist, kam mit iBOND^{™} Gluma Inside (Heraeus Kulzer) auf den Markt. Es kann als Nachteil betrachtet werden, dass iBOND'"" Gluma Inside kühl (bei 4-10°C) gelagert werden muss, da die Möglichkeit besteht, dass durch eine Hydrolyse der Bestandteile während der Lagerung bei höheren Temperaturen eine frühzeitige Polymerisation im Packmittel stattfindet.

Es wurden bereits verschiedene hydrolysestabile Monomere für Dentalmaterialien vorgeschlagen, darunter Sulfonsäurederivate mit (W003070198A1, US20030187094A1) und ohne Siloxangruppen (US20030055124A1) sowie Acrylesterphosphonsäuren und deren Ester, (US6710149B2, DE10206451A1, US6172131B1, US6812266B2), Amide (DE10101523A1, W003035013A1), oder Carbonsäurederivate mit PO-haltigen Gruppen (DE10242106A1).

Z.B ist in dem Produkt Adhese (Ivoclar Vivadent) ein hydrolysestabiles Phosphonsäureetheracrylat als saure Komponente zugesetzt.

Bei der Verwendung von selbstätzenden Dentaladhäsiven werden die Schritte der Konditionierung und des anschließende Applizierens des Adhäsivs (Bonding) in einem Schritt zusammengefasst. Ein vorheriges, separates Ätzen der Zahnhartsubstanz (Schmelz, Dentin) mit Phosphorsäure entfällt. Im Falle des Dentins lösen die säurehaltigen Adhäsivsysteme die Schmierschicht auf und legen das darunterliegende Dentin frei bzw. machen die Schmierschicht penetrierbar für das Adhäsiv. Simultan dazu findet die Infiltration der Monomere in die Zahnhartsubstanz statt. Im Falle des Schmelzes wird durch die säurehaltigen Adhäsivsysteme ein der Phosphorsäureätzung ähnliches Ätzmuster erzeugt. Abschließend wird das zum Ätzvorgang und zur Infiltration notwendige Lösungsmittel mit einem Luftpüster entfernt und das Adhäsiv strahlengehärtet.

Selbstkonditionierende Adhäsive enthalten als aktive Komponente saure, polymerisationsfähige Monomere. In der Regel handelt es sich bei diesen Monomeren um (Meth)acrylsäureester organischer oder anorganischer Säuren. Als Beispiele saurer Monomere seien Phosphorsäureester, wie HEMA-Phosphat, oder Ester der Trimellitsäure/des Trimellitsäureanhydrids genannt.

Aus der organischen Chemie ist bekannt, dass Ester in saurer, wässriger Lösung nicht hydrolysestabil sind. Dementsprechend kann es bei der Lagerung der erwähnten sauren Monomere in wässrigen Lösungen zur hydrolytischen Spaltung innerhalb weniger Wochen bis Monate kommen. So lässt sich zeigen, dass bei der Hydrolyse von HEMA-Phosphat die Verbindungen Methacrylsäure, HEMA und Phosphorsäure freigesetzt werden. Die Geschwindigkeit der Hydrolyse ist u.a. pH-Wert- und temperaturabhängig und läuft bei Kühlschranklagerung deutlich langsamer ab.

Es stellt sich die Aufgabe, hydrolysestabile Monomere bereitzustellen, die zur Herstellung selbstkonditionierender Adhäsive geeignet sind. In der Patentanmeldung EP 1 548 021 A1 publiziert a 29-6-2005 werden Verbindungen der Formel 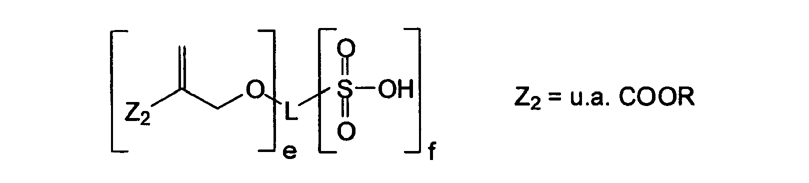 als Bestandteile einkomponentiger selbstätzender Dentaladhäsive vorgeschlagen.

Es wurde gefunden, dass Etherverbindungen organischer Säuren oder deren Anhydride einen weiteren Zugang zu hydrolysestabilen, sauren Monomeren mit ausgezeichneten Ätz- und Bonding-Eigenschaften - für sich allein oder in Kombination mit anderen sauren Monomeren - darstellen.

Die Erfindung betrifft Verbindungen der Formel I

R¹-O-CO-C(=CHR)-Y-O-Q(AH)ₙ (I)

worin A für eine -CO₂⁻ oder -SO₃⁻ -Gruppe steht, die zusammen mit H⁺ AH ergibt;
Q C₁₋₁₂Alkylen, durch -O- , >N- oder -S- unterbrochenes C₄₋₁₂Alkylen oder gegebenenfalls mit C₁₋₄Alkyl, C₁₋₄Alkoxy oder Halogen substituiertes C₆₋₁₅-Arylen darstellt, wobei im Fall von Arylen die Arylgruppen weitere Säuregruppen A tragen können;
Y für C₁₋₁₂Alkylen oder durch -O- , >N- oder -S- unterbrochenes C₄₋₁₂Alkylen steht;
R Methyl oder H darstellt;
R¹ C₁₋₆Alkyl ist; und
n die Werte 1,2 oder 3 annimmt
mit der Maßgabe, dass
Y C₂₋₁₂Alkylen ist, wenn A SO₃⁻ darstellt.

R¹ als C₁₋₆Alkyl bedeutet z.B. Methyl, Ethyl, n-Propyl, n-Butyl, n-Pentyl, n-Hexyl oder deren isomere Formen.

Y oder Q als C₁₋₁₂Alkylen stehen z.B. für Methylen, Ethylen, Propylen, Tetramethylen, Pentamethylen, 2,2-Dimethyltrimethylen, Hexamethylen, Heptamethylen, Octamethylen, Decamethylen oder Dodecamethylen.

Durch -O- , >N-oder -S- unterbrochene C₄₋₁₂Alkylengruppen, sind z.B. -CH₂-O-CH₂CH₂-O-CH₂-, -CH₂-(O-CH₂CH₂)₂-O-CH₂-, -CH₂-(O-CH₂CH₂)₃-O-CH₂-, -CH₂-(O-CH₂CH₂)₄-O-CH₂- und insbesondere -CH₂CH₂-O-CH₂CH₂-O-CH₂CH₂-, -CH₂-CH₂-NMe-CH₂CH₂- oder -CH₂CH₂-S-CH₂CH₂-, worin Me Methyl ist.

C₆₋₁₅Arylen kann z.B. sein: o-, m- oder p-Phenylen, 1,4-Naphthylen und 4,4'-Diphenylen.
mit C₁₋₄Alkyl, C₁₋₄Alkoxy oder Halogen substituiertes C₆₋₁₅Arylen sind z.B die oben genannten o-, m- oder p-Phenylen, 1,4-Naphthylen und 4,4'-Diphenylen, die am Ring mit -CH₃, -C₂H₅, - OCH₃ oder Cl substituiert sind.

Die Verbindungen der Formel I können insbesondere als Bestandteil von Adhäsiven, Zementen, Kompositen und Formkörpern sowie bevorzugt von Dentalmaterialien verwendet werden. Dabei ist es möglich, dass sie in zumindest teilweise polymerisierter Form vorliegen.

Bei Verwendung des hydrolysestabilen, sauren, polymerisierbaren Monomers der Formel I in einer Adhäsivformulierung wird die Lagerstabilität verbessert. In Kombination mit weiteren hydrolysestabilen und polymerisierbaren Molekülen ist eine Kühllagerung zur Gewährleistung der Langzeitstabilität nicht mehr notwendig.

Die Verbindungen der Formel I eignen sich besonders für Dentaladhäsive zur Befestigung direkter Füllungswerkstoffe wie Komposite, Compomere und Ormocere.

Entsprechend betrifft die Erfindung auch Zusammensetzungen enthaltend
A mindestens eine Verbindung der Formel I,
B mindestens ein weiteres polymerisierbares Monomer,
C einen oder mehre Initiatoren sowie gegebenenfalls Stoffe aus den Gruppen der
D Füllstoffe, Pigmente Stabilisatoren, UV-Absorber, Farbstoffe oder Gleitmittel.

Vorteilhaft können solche Adhäsive in Verbindung mit einer zusätzlichen, die sauren Bestandteile abdeckenden oder neutralisierenden Schicht und einem Befestigungszement zur Befestigung indirekter, laborgefertigter Füllungswerkstoffe aus z.B. Keramik oder Komposit eingesetzt werden.

Die Verbindungen der Formel I eignen sich ferner als Additive in Fissurenversieglern oder Ästhetik-Lacken.

Polymerisierbare Monomere, Initiatoren, Füllstoffe, Pigmente und Stabilisatoren sind dem Dentalfachmann bekannt.

Als herkömmliche, radikalisch polymerisierbare Monomere eignen sich insbesondere difunktionelle Vernetzermonomere. Für die Herstellung von Adhäsiven oder Dentalmaterialien eignen sich vor allem vernetzende bi- oder mehrfunktionelle Acrylate oder Methacrylate, wie z. B. Bisphenol-A- di(meth)acrylat, als Bis-GMA bezeichnetes Additionsprodukt von Methacrylsäure und Bisphenol-A-diglycidylether, als UDMA bezeichnetes Additionsprodukt von Hydroxyethylmethacrylat und 2,2,4-Trimethylhexamethylendiisocyanat, Di-, Tri- oder Tetraethylenglykol-di(meth)acrylat, Decandioldi(meth)acrylat, Trimethylolpropantri(meth)acrylat und Pentaerythrit-tetra(meth)acrylat. Es eignen sich ebenfalls die durch Veresterung von (Meth)acrylsäure mit den entsprechenden Diolen zugänglichen Verbindungen Butandioldi(meth)acrylat, 1,10-Decandioldi(meth)acrylat und 1,12- Dodecandioldi(meth)acrylat.

Darüber hinaus können die erfindungsgemäßen Zusammensetzungen zur Verbesserung der mechanischen Eigenschaften mit organischen oder anorganischen Partikeln oder Fasern gefüllt werden. Bevorzugte anorganische partikuläre Füllstoffe sind amorphe kugelförmige Materialien auf der Basis von Mischoxiden aus SiO2, Zr02 und/oder Ti02, mikrofeine Füllstoffe, wie pyrogene Kieselsäure oder Fällungskieselsäure, sowie Makro-, Mikro- oder Nanofüllstoffe, wie Quarz-, Glaskeramik- oder Glaspulver mit einer durchschnittlichen Teilchengröße von 0,001 bis 5 µm. Schließlich können auch röntgenopake Füllstoffe, wie Ytterbiumtrifluorid, oder Glasfasern, Polyamid- oder Kohlenstoff-Fasern eingesetzt werden.

Die erfindungsgemäßen Zusammensetzungen können im Bedarfsfall weitere Komponenten enthalten, vor allem Lösungsmittel, wie Wasser, Ethylacetat, Aceton, Ethanol oder Mischungen von diesen, sowie Stabilisatoren, UV-Absorber, Farbstoffe, Pigmente oder Gleitmittel.

Die Verbindungen der Formel I eignen sich besonders als Bestandteil von Dentaladhäsiven und Mitteln zum Ätzen und Bonden von Dentin oder Zahnschmelz, aber auch von weiteren Dentalmaterialien wie Befestigungszementen, Restaurationscompositen, Unterfüllungsmaterialien, Dental-Lacken, Fissurenversieglern. Solche Materialien zeichnen sich durch eine sehr gute Haftung auf unterschiedlichen Substraten, wie der Zahnhartsubstanz und metallischen Substraten, aus. Entsprechend betrifft die Erfindung auch solche - Verbindungen der Formel I enthaltende Materialien sowie die Verwendung von Verbindungen der Formel I zu deren Herstellung.

Die Hydrolysestabilität der Verbindungen der Formel I verleiht dabei den erfindungsgemässen Materialien ebenfalls eine sehr gute Hydrolysestabilität. Das gilt sowohl für das unpolymerisierte als auch das polymerisierte Material. Eine hohe Hydrolysestabilität ist naturgemäß für solche Materialien von besonderer Bedeutung, die permanent wässrigen Medien ausgesetzt sind, wie dies gerade bei Dentalmaterialien der Fall ist, die für einen längere Verbleib in der Mundhöhle vorgesehen sind.

Zur Durchführung der Polymerisation können die bekannten radikalischen Initiatoren (vgl. Encyclopedia of Polymer Science and Engineering, Vol. 13, Wiley-Interscience Publisher, New York 1988, 754 ff.) eingesetzt werden. Es eignen sich besonders Azoverbindungen, wie Azobis(isobutyronitril) (AIBN) oder Azobis- (4-cyanvaleriansäure) oder Peroxide, wie Dibenzoylperoxid, Dilauroylperoxid, tert.-Butylperoctoat, tert.-Butylperbenzoat oder Di-(tert.-butyl)-peroxid.

Als Initiatoren für die Heißhärtung eignen sich auch Benzpinakol und 2,2'-Dialkylbenzpinakole.

Weiterhin können auch Photoinitiatoren (vgl. J. P. Fouassier, J. F. Rabek (Hrsg.), Radiation Curing in Polymer Science and Technology, Vol. II, Elsevier Applied Science, London und New York 1993) für die Polymerisation mit UV-Licht oder Licht sichtbarer Wellenlängen, wie Benzoinether, Dialkylbenzilketale, Dialkoxyacetophenone, Acylphosphinoxide, alpha -Diketone, wie 9,10- Phenanthrenchinon, Diacetyl, Furil, Anisil, 4,4'-Dichlorbenzil und 4,4'-Dialkoxybenzil, und Campherchinon, verwendet werden.

Die Herstellung der Verbindungen der Formel I erfolgt nach an sich bekannten Verfahren, z.B. indem man
eine Verbindung der Formel II

R₁-O-CO-C(=CHR)Y-Br (II)

mit einer Verbindung der Formel III

HO-Q(AL)ₙ (III)

umsetzt, wobei L eine Schutzgruppe darstellt, welche die Protonen ersetzt.

Vorzugsweise wird die Reaktion in einem aprotischen Lösungsmittel durchgeführt, bevorzugt in Dimethylformamid (DMF). Begonnen wird vorteilhaft bei Raumtemperatur. Exothermie kann Kühlung erforderlich machen.

Das folgende Beispiel erläutert eine Ausführungsform der Erfindung, ohne die Erfindung darauf zu beschränken (Prozentangaben beziehen sich auf das Gewicht, sofern nicht anders vermerkt):

### Beispiel 1: 4-(2-Ethoxycarbonyl-allyloxy)-phthalsäure

### Vor-Vorprodukt A1: "5-Hydroxy-1,2-Dicarbonsäure"

Ausgangsmaterialien: 100 ml 4-Sulfophthalsäure techn. 50% in Wasser (d = 1.292); 131.4 g NaOH-Plätzchen

Die 4-Sulfo-phthalsäurelösung wird in einem Stahlbecher vorsichtig mit den ersten 30 g NaOH-Plätzchen versetzt. Dabei erhitzt sich die Mischung bis zum Siedepunkt. Zwei zugesetzte Siedesteine verhindern größere Siedeverzüge. Man taucht den Becher in das auf 210°C vorgeheizte Ölbad ein und setzt unter Rühren mit einem Spatel die restlichen NaOH-Plätzchen in Portionen zu. Während des Eintragens wird immer wieder etwas Aufschäumen beobachtet, welches sich durch Rühren und zeitweiliges kurzes Absenken des Bades unter Kontrolle halten lässt. Nach vollständiger Zugabe binnen etwa 30 Minuten senkt man die Badtemperatur auf 200°C und lässt die Suspension unter zeitweiligem Bewegen mit dem Spatel noch während 2h reagieren.

Die Suspension wird nun auf ein Stahlblech ausgegossen und während des Erstarrens mit dem Spatel aufgekratzt, um eine größere Oberfläche zu erhalten.

Mit insgesamt 1,5 Liter Wasser wird aufgelöst und in ein Becherglas gegeben. Unter Eiskühlung von außen wird mit 340 ml HCl conc. sauer gestellt. Die Temperatur lässt man währenddessen zeitweilig bis auf 50°C ansteigen. Nach dem Ansäuern erhält man ca. zwei Liter einer fast farblosen klaren Lösung.

Die Lösung wird mit einmal 500 ml und zweimal 300 ml Essigester extrahiert. Die organischen Phasen werden zweimal mit 150 ml verdünnter und einmal mit 100 ml conc. Sole gewaschen. Nach Trocknen über MgS04 wird filtriert, eingedampft und am Rotavapor getrocknet. Es ergeben sich als Rohprodukt 42,2 g farblose Kristalle (86 %). Das Rohprodukt wird zusammen mit dem Rohprodukt eines weiteren Ansatzes (40,6 g) mit 400 ml Essigester während 30 Minuten zum Rückfluss erhitzt. Nach Abkühlen auf RT wird über Nacht stehen gelassen. Die Suspension wird im Eisbad gekühlt, filtriert und mit eiskaltem Essigester gewaschen.

Nach Filtrieren wird im Trockenschrank am Hausvakuum bei 50°C getrocknet. Umkristallisation aus Essigester ergibt 68.26 g farblose Kristalle, Fp:202-203 °C, Ausbeute 71,5% bezogen auf 0.524 Mol.

HPLC und NMR ergeben ein isomerenfreies Produkt.

### Vorprodukt A: 5-Hydroxyphthalsäureanhydrid

Der Kolben mit dem Produkt der Stufe 1 wird in ein auf ca. 200°C vorgeheiztes Ölbad eingetaucht. Nach einigen Minuten färben sich die vorher farblosen Kristalle bräunlich um dann unter leichtem Schäumen in eine hellbraune Schmelze überzugehen. Nach 30 Minuten wird der Kolben aus dem Bad gehoben. Es tritt sofort wieder Kristallisation ein.

Das Rohgewicht betrug nun 0.39 g. Zum NMR, HPLC und LC-MS als SO1081.015 roh. Fp: ab 162°C sintern, 164-169°C schmelzen.

Das Produkt soll mit einem späteren Ansatz zusammen umkristallisiert werden.

### Vor-Vorprodukt B1 2-Hydroxvmethyl-Acrylsäureethylester

16.2 ml CH2O-Lösung (37%) in Wasser werden mit 65,2 ml Acrylsäureethylester, 1800 ml Dioxan und 22.4g DABCO vereinigt und bei RT gerührt. Nach 14 Stunden wird via HPLC der Fortschritt der Umsetzung geprüft. Es wurde zunächst am Rotavapor bei ca. 40°C Badtemperatur und <70 mbar Druck beginnend das Lösungsmittel abdestilliert.

Dabei destilliert auch der Überschuss Acrylester ab. Die zurückbleibende klare wässrige Lösung wird im Scheidetrichter dreimal mit Methyl-tert.butylether extrahiert. Die Extrakte werden zweimal mit Sole gewaschen, über Mg-Sulfat getrocknet, filtriert und eingedampft.

Es ergeben sich als Rückstand 17,3 g (66,5 %) klares, farbloses Öl, das laut NMR noch ca. 20 Mol % Dioxan enthält.

vgl. Literatur: Chengzhi Yu et al. J. Org. Chem. 2001, 66 5413-5418.

### Vorprodukt B 2-Bromomethyl-Acrylsäureethylester

10 g 2-Hydroxymethyl-Acrylsäureethylester werden in Ether vorgelegt, auf - 15°C abgekühlt, und 3.2 ml (9.26 g) PBr3 werden binnen 5 Min zugetropft. Die Kühlung wird entfernt und 2 Stunden bei RT gerührt.

Aus der farblosen Lösung hat sich ganz wenig eines weißen Niederschlages abgeschieden. Es wird wieder auf -15°C abgekühlt und 100 ml Wasser zugetropft. Anfänglich war dies exotherm unter etwas Gasentwicklung, nachdem aber ca. 10 ml zugetropft sind, kann man den Rest schnell zulaufen lassen. Die Phasen werden getrennt und die wässrige Phase extrahiert mit Diethylether (3x50 ml), die vereinigten organische Phasen zweimal mit Wasser und einmal mit Sole gewaschen, mit M9S04 getrocknet filtriert und eingedampft. Es ergeben sich 13.67 g klare leicht gelbliche Flüssigkeit.

### 4-(2-Ethoxycarbonyl-allyloxy)-phthalsäure

Die Vorprodukte A (11.55g) und B (13,67 g) werden in 100 ml DMF unter Rühren vorgelegt und bei RT 4,65 g Pottasche zugegeben. Man bemerkt sofort eine Gelbfärbung der Suspension und eine leichte Gasentwicklung. Die Reaktion ist schwach exotherm. Man stellt vorsichtshalber ein kaltes Wasserbad als Kühlung unter den Kolben. Es wird bei RT 2 h rühren gelassen. Nach Aufarbeitung erhält man trocken 20.52 g farblosen kristallinen Rückstand.

Das Rohprodukt wird mit 80 ml Essigester kochend gelöst und die gelbliche Lösung filtriert. Anschließend wird am Rotavapor bei ca. 200 mbar so viel Essigester abgezogen, dass ein Endgewicht der Lösung von ca. 61 g resultiert. Dies entspricht noch einem Essigesteranteil von ca. 47 g. Beim Animpfen beginnt das Produkt in farblosen, sehr feinen Kristallnädelchen auszukristallisieren. Es wird mit 20 ml Ether versetzt und im Eisbad gekühlt. Nach Filtrieren wird mit eiskalter Essigester/Ether Mischung gewaschen und im Vakuumtrockenschrank bei 40°C getrocknet.

Ausbeute 10.52 g ( 50.8 %) farblose Kristalle

Fp:-147°C

Die Mutterlauge wird eingedampft und der wieder kristallin gewordene Rückstand mit Ether digeriert. Nach 3 Stunden wird bei RT filtriert und mit Ether gewaschen. Nach Trocknen im Vakuum ergeben sich als zweite Fraktion 2.54 g (12.2 %) farblose Kristalle.

### Beispiel 2 A-D: Test der Wirksamkeit als saures Monomer in einer Adhäsivformulierung

Die folgenden Zubereitungen werden durch intensives Vermischen der Bestandteile erzeugt:

| **Beispiel 2** | **A** | **B** | **C** | **D** |
|---|---|---|---|---|
| Urethandimethacrylat | 10 | 15 | 20 | 25 |
| 4-(2-Ethoxycarbonyl-allyloxy)-phthalsäure | 20 | 15 | 10 | 5 |
| Aceton | 40 | 40 | 40 | 40 |
| Wasser | 30 | 30 | 30 | 30 |
| Campherchinon | 0,2 | 0,2 | 0,2 | 0,2 |
| 2-n-Butoxyethyl-4-(dimethylamino)benzoat | 0,3 | 0,3 | 0,3 | 0,3 |

Die Wirksamkeit als saures Monomer in einer Adhäsivformulierung wird geprüft durch Bestimmung der Scherbindungsfestigkeit auf Dentin und Schmelz. Es werden menschliche Zähne benutzt, die für max. drei Monate nach der Extraktion in 0,5 % Chloramin T-Lösung aufbewahrt worden sind. Vor der Verwendung im Bindungstest werden die Zähne sorgfältig unter fließendem Wasser gereinigt. Am Tage vor der Verwendung im Bindungstest werden die Zähne einzeln auf einer Approximalseite liegend mit Technovit 4001 (schnellhärtender Kunststoff auf Methylmethacrylat-Basis, Fa. Heraeus-Kulzer) in zylindrischen Gummiformen eingebettet. Die Zähne werden durch Nassschleifen mit SiC-Papieren der Körnungen 80, 240 und schließlich 600 soweit beschliffen, dass eine ausreichend große Dentin- bzw. Schmelzfläche zur Anbindung eines Kunststoffzylinders mit 3,5 mm Durchmesser freiliegt. Nach Abspülen mit entsalztem Wasser werden die Zähne im Luftstrom getrocknet. Auf die Zahnoberfläche werden die Zubereitungen aus den Beispielen 2 A-D mit einem Pinsel in drei Schichten aufgetragen, im Druckluftstrom getrocknet und mit dem Lichtgerät Translux^{®} Energy (Halogenlicht,_Heraeus Kulzer) 20 Sekunden lang bestrahlt. Die so vorbehandelte Probe wird dann mittels einer Einspannvorrichtung unter eine zweigeteilte zylindrische Teflonform (3,5 mm Durchmesser, 1 mm Höhe) festgeklemmt. Danach wird das Kunststoff-Füllungsmaterial Charisma^{®} (lichthärtendes Composit auf Methacrylat Basis, Fa. Heraeus Kulzer) in die Teflonform eingefüllt, mit einer Sauerstoff-undurchlässigen PE-Folie abgedeckt und mit dem Lichtgerät Translux^{®} Energy (Heraeus Kulzer) 20 Sekunden lang bestrahlt. Unmittelbar anschließend wird die Teflonform abgenommen und die zylindrische Probe für 24 h in 37°C warmen Wasser gelagert bis zur Einleitung der Scherbelastung. Dazu wird die zylindrische Probe in einer Universalprüfmaschine mit Hilfe eines Druckstempels parallel zu und dicht an der angeschliffenen Zahnoberfläche bei einer Geschwindigkeit von 1 mm/min bis zur Trennung des KunststoffZylinders vom Zahn belastet. Die Scherbindungsfestigkeit ist der Quotient aus Bruchkraft und Bindungsareal und wird jeweils an 8 Proben bestimmt, wobei deren Mittelwert in der Tabelle angegeben ist.

Ergebnisse:

| **Zubereitung** | **Scherbindungsfestigkeit an Dentin [MPa]** | **Scherbindungsfestigkeit an Schmelz [MPa]** |
|---|---|---|
| 2A | 17,0 | 6,4 |
| 2B | 17,1 | 7,0 |
| 2C | 24,6 | 9,8 |
| 2D | 12,9 | 6,2 |

Die Messung des Scherhaftverbunds an Dentin und Schmelz bestätigt, dass die Verbindung der Formel I, 4-(2-Ethoxycarbonyl-allyloxy)-phthalsäure, als saures Monomer in Adhäsiven wirksam ist.

## Patentansprüche

1. Verbindungen der Formel I
R¹-O-CO-C(=CHR)-Y-O-Q(AH)ₙ (I)
worin A für eine -CO₂⁻ oder -SO₃⁻ -Gruppe steht, die zusammen mit H⁺ AH ergibt;
Q C₁₋₁₂Alkylen, durch -O-, >N- oder S- unterbrochenes C₄₋₁₂Alkylen oder gegebenenfalls mit C₁₋₄Alkyl, C₁₋₄Alkoxy oder Halogen substituiertes C₆₋₁₅Arylen darstellt, wobei im Fall von Arylen die Arylgruppen weitere Säuregruppen A tragen können;
Y für C₁₋₁₂Alkylen oder durch -O- , >N- oder -S- unterbrochenes C₄₋₁₂Alkylen steht;
R Methyl oder H darstellt;
R¹ C₁₋₆Alkyl ist; und
n die Werte 1,2 oder 3 annimmt
mit der Maßgabe, dass
Y C₂₋₁₂Alkylen ist, wenn A SO₃⁻ darstellt.

2. Verfahren zur Herstellung von Verbindungen der Formel I gemäß Anspruch 1, **dadurch gekennzeichnet, dass**
eine Verbindung der Formel II
R₁-O-CO-C(=CHR)Y-Br (II)
mit einer Verbindung der Formel III
HO-Q(AL)ₙ (III)
umgesetzt wird, wobei L eine Schutzgruppe darstellt, welche die Protonen ersetzt.

3. Zusammensetzungen enthaltend
A mindestens eine Verbindung der Formel I gemäß Anspruch 1
B mindestens ein weiteres polymerisierbares Monomer
C einen oder mehre Initiatoren sowie gegebenenfalls
D Füllstoffe und/oder Pigmente und /oder Stabilisatoren.

4. Dentalmaterialien enthaltend mindestens eine Verbindung der Formel I gemäß Anspruch 1.

5. Dentalmaterialien gemäß Anspruch 4 in Form von Befestigungszementen, Restaurationscompositen, Unterfüllungsmaterialien, Dental-Lacken, Fissurenversieglern, Dentaladhäsiven oder Mitteln zum Ätzen und Bonden von Dentin oder Zahnschmelz.

6. Verwendung von Verbindungen der Formel I gemäß Anspruch 1 zur Herstellung von Befestigungszementen, Restaurationscompositen, Unterfüllungsmaterialien, Dental-Lacken, Fissurenversieglern, Dentaladhäsiven oder Mitteln zum Ätzen und Bonden von Dentin oder Zahnschmelz.

## Claims

1. Compounds of formula I
R¹-O-CO-C(=CHR)-Y-O-Q(AH)ₙ (I)
where A represents a -CO₂⁻ or -SO₃⁻ group, which results in AH when it is combined with H⁺;
Q represents C₁₋₁₂alkylene, C₄₋₁₂alkylene interrupted by -O-, >N- or -S-, or, if applicable, C₁₋₄alkyl-, C₁₋₄alkoxy- or halogen-substituted C₆₋₁₅₋arylene, whereby, in the case of arylene, additional acid groups can bear A;
Y represents C₁₋₁₂alkylene or C₄₋₁₂alkylene interrupted by -O-, >N- or -S-;
R represents methyl or H;
R¹ is C₁₋₆alkyl; and
n is 1, 2 or 3
on the condition that
Y is C₂₋₁₂alkylene if A represents SO₃⁻.

2. Method for the production of compounds of formula I according to claim 1, **characterized in that**
a compound of formula II
R₁-O-CO-C(=CHR)Y-Br (II)
is reacted with a compound of formula III
HO-Q(AL)ₙ (III),
whereby L is a protective group replacing the protons.

3. Compositions containing
A at least one compound of formula I according to claim 1
B at least one more polymerizable monomer
C one or more initiators, and, if applicable,
D fillers and/or pigments and /or stabilizers.

4. Dental materials containing at least one compound of formula I according to claim 1.

5. Dental materials according to claim 4 in the form of fixation cements, restoration composites, base lining materials, dental varnishes, fissure sealants, dental adhesives or agents for etching and bonding of dentine or enamel.

6. Use of compounds of formula I according to claim 1 for the production of fixation cements, restoration composites, base lining materials, dental varnishes, fissure sealants, dental adhesives or agents for etching and bonding of dentine or enamel.

## Revendications

1. Composés de la formule 1
R¹-O-CO-C(=CHR)-Y-O-Q(AH)ₙ (I),
dans laquelle A représente un groupe -CO₂⁻ ou -SO₃⁻ qui donne AH avec H⁺;
Q représente alkylène en C₁₋₁₂, alkylène en C₄₋₁₂ interrompu par -O-, >N- ou -S- ou arylène en C₆₋₁₅ éventuellement substitué par alkyle en C₁₋₄, alcoxy en C₁₋₄ ou halogène, les groupes aryle pouvant porter d'autres groupes acide A dans le cas de l'arylène ;
Y représente alkylène en C₁₋₁₂ ou alkylène en C₄₋₁₂ interrompu par -O-, >N- ou -S- ;
R représente méthyle ou H ;
R¹ est alkyle en C₁₋₆ ; et
n prend les valeurs 1, 2 ou 3,
à condition que
Y soit alkylène en C₂₋₁₂ lorsque A représente SO₃⁻.

2. Procédé de fabrication de composés de la formule 1 selon la revendication 1, **caractérisé en ce que**
un composé de la formule II
R₁-O-CO-C(=CHR)Y-Br (II)
est transformé avec un composé de la formule III
HO-Q(AL)ₙ (III),
L représentant un groupe protecteur qui remplace les protons.

3. Compositions contenant
A au moins un composé de la formule I selon la revendication 1,
B au moins un autre monomère polymérisable,
C un ou plusieurs initiateurs, ainsi qu'éventuellement
D des charges et/ou pigments et/ou stabilisants.

4. Matériaux dentaires contenant au moins un composé de la formule I selon la revendication 1.

5. Matériaux dentaires selon la revendication 4 sous la forme de ciments de fixation, composites de restauration, matériaux de comblement, vernis dentaires, agents de scellement de fissures, adhésifs dentaires ou moyens de graver et relier de la dentine ou de l'émail dentaire.

6. Utilisation de composés de la formule I selon la revendication 1 pour la fabrication de ciments de fixation, composites de restauration, matériaux de comblement, vernis dentaires, agents de scellement de fissures, adhésifs dentaires ou moyens de graver et relier de la dentine ou de l'émail dentaire.
